# EUROPEAN PATENT SPECIFICATION

(11) **EP 4 255 377 B1**
(45) Date of publication and mention of the grant of the patent: **10.04.2024**
(21) Application number: 21806250.3
(22) Date of filing: 09.11.2021
(51) Int. Cl.: A61K 8/34, A61K 8/44, A61K 8/60, A61Q 19/10

(54) **PERSONAL CARE COMPOSITION COMPRISING GLYCINATE SURFACTANT, POLYOL AND NONIONIC SURFACTANT COMPRISING ALKYL GLUCOSIDE**
KÖRPERPFLEGEZUSAMMENSETZUNG, DIE EIN GLYCINAT-TENSID, EIN POLYOL UND EIN NICHTIONISCHES TENSID MIT ALKYLGLUCOSID ENTHÄLT
COMPOSITION DE SOINS PERSONNELS COMPRENANT UN AGENT TENSIOACTIF GLYCINATE, UN POLYOL ET UN AGENT TENSIOACTIF NON IONIQUE COMPRENANT DU GLUCOSIDE D'ALKYL

(30) Priority: 02.12.2020 WO PCT/CN2020/133390; 15.01.2021 EP 21151719
(43) Date of publication of application: 11.10.2023
(73) Proprietor: Unilever IP Holdings B.V., 3013 AL Rotterdam (NL); Unilever Global IP Limited, Wirral, Merseyside CH62 4ZD (GB)
(72) Inventor: LIU, Lanhua, Shanghai 200335 (CN); PAN, Chanchan, Shanghai 200335 (CN)
(74) Representative: James, Helen Sarah
(86) International application number: PCT/EP2021/081013
(87) International publication number: WO 2022/117290

(56) References cited:
- WO-A1-2019/127001
- WO-A1-2019/127089
- CN-A- 106 883 959
- CN-A- 108 379 143
- CN-A- 108 542 814
- KR-A- 20090 069 958
- KR-A- 20200 014 154
- US-A1- 2006 183 662
- DATABASE GNPD [Online] MINTEL; 21 October 2020 (2020-10-21), anonymous: "Foaming Hand Soap", XP055819611, Database accession no. 8204425

## Description

### Field of the Invention

The present invention relates to a personal care composition suitable for use in the automatic dispensing device. In particular such composition is capable of generating less residue when it is pumped out as foam and the foam is dried, reducing the risk of blocking the nozzle of the device.

### Background of the Invention

Automatic dispensing device has been widely used to provide personal care composition, for example skin cleansing composition, on many occasions due to the convenience and the contactless mode of such device. The development of personal care composition suitable for automatic dispensing device is therefore critical for consumer use. Amino acid-based surfactant is a typical mild surfactant and therefore it is desirable to be incorporated into such composition.

However, the present inventors surprisingly found that personal care composition containing glycinate surfactant generated residues when such composition is pumped out from the device as foam and such foam is dried. Thus, the risk of blocking nozzle is high if such composition is employed in the automatic dispensing device. The present inventors have recognized the needs to develop a personal care composition containing glycinate surfactant has less residue when it is dried. It was surprisingly found that by combining nonionic surfactant and including polyol with certain level. The generated residues were significantly reduced.

### Summary of the Invention

In a first aspect, the present invention is directed to personal care composition comprising glycinate surfactant, nonionic surfactant comprising alkyl glucoside, and polyol, wherein the weight ratio of the polyol to the glycinate surfactant is no less than 5.4:1; the composition comprises phenoxyethanol; and the total amount of the glycinate surfactant and alkyl glucoside is at least 2% by weight of the composition.

In a second aspect, the present invention is directed to use of polyol to reduce the dried residue of a composition containing glycinate surfactant, wherein the weight ratio of the polyol to the glycinate surfactant is no less than 5.4:1.

All other aspects of the present invention will more readily become apparent upon considering the detailed description and examples which follow.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of". In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) mutatis mutandis.

Preferably the glycinate surfactant comprises alkyl glycinate, acyl glycinate (also known as alkyl carboxy glycinate or alkanoyl glycinate) or a mixture thereof. More preferably, the glycinate surfactant comprises C₈₋₂₄ alkyl glycinate, C₈₋₂₄ acyl glycinate, or a mixture thereof. Even more preferably, the glycinate surfactant comprises C₁₂₋₂₀ alkyl glycinate, C₁₂₋₂₀ acyl glycinate, or a mixture thereof. It should be noted that the glycinate surfactant is different from the nonionic surfactant. Preferably, the glycinate surfactant is anionic.

Preferably, the glycinate surfactant is acyl glycinate, more preferably C₈₋₂₄ acyl glycinate, even more preferably C₁₂₋₂₀ acyl glycinate and still even more preferably cocoyl glycinate and most preferably sodium cocoyl glycinate.

The glycinates are generally used in the form of their salts. Preferably, the glycinate is in the form of alkali metal salt, and/or ammonium salt. More preferably, the glycinate is in the form of potassium, sodium salt and/or trialkanolammonium salt.

Typically, the glycinate surfactant is present in amount of 0.001 to 15% by weight of the composition. Preferably, the glycinate surfactant is present in amount of 0.1 to 10%, more preferably 0.5 to 6%, even more preferably 1.5 to 4% by weight of the composition.

Polyol is a term used herein to designate a compound having at least two hydroxyl groups. Preferably, the polyol has 2 to 20 carbon atoms, more preferably 2 to 10 carbon atoms, and most preferably 2 to 4 carbon atoms. Preferably, the polyhydric alcohol has a molecular weight of 50 to 500, more preferably 60 to 300.

Typically, the polyol comprises glycerin (i.e., glycerine or glycerol), propylene glycol, dipropylene glycol, polypropylene glycol, polyethylene glycol, sorbitol, hydroxypropyl sorbitol, hexylene glycol, 1,3-butylene glycol, isoprene glycol, 1,2,6-hexanetriol, ethoxylated glycerol, propoxylated glycerol or a mixture thereof. Preferably, the polyol comprises glycerin, propylene glycol, butylene glycol, polypropylene glycol, polyethylene glycol, sorbitol or a mixture thereof. More preferably, the polyol comprises glycerin, propylene glycol, butylene glycol, sorbitol or a mixture thereof. Even more preferably the polyol comprises glycerin, propylene glycol, butylene glycol, or a mixture thereof. Most preferably, the polyhydric alcohol comprises glycerin.

Typically, the polyol is present in amount of 0.1 to 50% by weight of the composition. Preferably, the polyol is present in amount of 4 to 45%, more preferably 6 to 40%, even more preferably 12 to 35%, still even more preferably 16 to 30% and most preferably 18 to 25% by weight of the composition. Preferably, the composition comprises glycerin in amount of in amount of 4 to 45%, more preferably 6 to 40%, even more preferably 12 to 35%, still even more preferably 16 to 30% and most preferably 18 to 25% by weight of the composition, when the glycerin is present.

The weight ratio of the polyol to the glycinate surfactant is preferably at least 6.3:1, more preferably at least 7:1. Preferably the weight ratio of the polyol to the glycinate surfactant is preferably less than 200:1, more preferably less than 50:1. The weight ratio of the glycerin to the glycinate surfactant is generally at least 5.4:1, preferably at least 6.3:1, more preferably at least 7:1. Preferably the weight ratio of the glycerin to the glycinate surfactant is preferably less than 200:1, more preferably less than 50:1, if the glycerin is present.

The nonionic surfactant comprises alkyl glucoside, more preferably C₆ to C₂₀ alkyl glucoside, even more preferably the nonionic surfactant comprises nonionic surfactant having INCI (International Nomenclature of Cosmetic Ingredients) name of decyl glucoside. Alkyl glucoside as used herein includes alkyl polyglucosides.

Preferred alkyl glucosides is represented by formula of RO - (G)ₙ, wherein R is a branched or straight chain alkyl group which may be saturated or unsaturated, G is a saccharide group, and the degree of polymerisation, n, may have a value of from 1 to 10; Preferably R has a mean alkyl chain length of from C₅ to C₂₀, G is selected from C₅ or C₆ monosaccharide residues and n has a value of from 1 to 6; more preferably R has a mean alkyl chain length of from C₆ to C₁₆, G is glucose and n has a value of from 1 to 6. Even more preferably, R has a mean alkyl chain length of from C₆ to C₁₆, G is glucose and n has a value of from 1 to 6.

Typically, the nonionic surfactant is present in amount of 0.001 to 15% by weight of the composition. Preferably, the nonionic surfactant is present in amount of 0.1 to 10%, more preferably 0.5 to 6%, even more preferably 1.5 to 4% by weight of the composition.

Typically, the alkyl glucoside is present in amount of at least 0.01%, preferably at least 0.1%, more preferably at least 0.5%, even more preferably at least 1%, still even more preferably at least 1.5% by weight of the composition. Typically, the alkyl glucoside is present in amount of no greater than 15%, preferably no greater than 12%, more preferably no greater than 8%, even more preferably no greater than 6%, still even more preferably at least 6% by weight of the composition.

To achieve desirable mildness and cleansing capability, the weight ratio of the glycinate surfactant to the nonionic surfactant is preferably in the range of 1:20 to 20:1, more preferably 1:8 to 8:1, even more preferably 1:4 to 4:1, and most preferably 1:2 to 2:1. The weight ratio of the glycinate surfactant to the alkyl glucoside is preferably in the range of 1:20 to 20:1, more preferably 1:8 to 8:1, even more preferably 1:4 to 4:1, and most preferably 1:2 to 2:1, if the alkyl glucoside is present.

The total amount of the glycinate surfactant and alkyl glucoside is at least 2% by weight of the composition. More preferably, the total amount of the glycinate surfactant and alkyl glucoside is 2 to 20%, even more preferably 2 to 15%, still even more preferably 3 to 10% and most preferably 4 to 8% by weight of the composition.

The composition may additionally comprise amino acid-based surfactant other than glycinate for example glutamate, in particular acyl glutamate, more preferably C₈ to C₂₀ acyl glutamate. Amount of glutamate may be in the range of 0.1 to 20%, preferably 1 to 10% by weight of the composition.

The composition preferably additionally comprises an amphoteric surfactant preferably a betaine surfactant preferably an alkyl amidopropyl betaine surfactant for example cocamidopropyl betaine (CAPB). Preferably, the composition comprises from 0.1 to 10%, preferably from 0.5 to 8%, more preferably from 1 to 5% of a betaine surfactant by weight of the composition.

For sake of mildness, sulfate containing anionic surfactants, such as SLES (Sodium Lauryl Ether Sulphate), SLS (Sodium Lauryl Sulfate), and soaps and blends thereof are present at maximum concentration levels of 3, 2 or 1% by weight of the composition, and are preferably absent from the composition.

Water-insoluble skin benefit agents may also be formulated into the compositions as conditioners and moisturizers. Examples include silicone oils; hydrocarbons such as liquid paraffins, petrolatum, microcrystalline wax, and mineral oil; and vegetable triglycerides such as sunflower seed and cottonseed oils.

Some compositions may include thickeners. These may be selected from cellulosics, natural gums and acrylic polymers but not limited by this thickening agent types. Among the cellulosics are sodium carboxymethyl cellulose, hydroxyethyl cellulose, hydroxypropyl methylcellulose and combinations thereof. Suitable gums include xanthan, pectin, karaya, agar, alginate gums and combinations thereof. Among the acrylic thickeners are homopolymers and copolymers of acrylic and methacrylic acids including carbomers such as Carbopol 1382, Carbopol 982, Ultrez, Aqua SF-1 and Aqua SF-2 available from the Lubrizol Corporation. Amounts of thickener may range from 0.01 to 3% by weight of the active polymer (outside of solvent or water) in the compositions.

Preservatives can desirably be incorporated into the compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatabilities between the preservatives and other ingredients. Preservatives are preferably employed in amounts ranging from 0.01% to 2% by weight of the composition.

A variety of other optional materials may be formulated into the compositions. These may include: antimicrobials such as 2-hydroxy-4,2',4'-trichlorodiphenylether (triclosan), 2,6-dimethyl-4-hydroxychlorobenzene, and 3,4,4'-trichlorocarbanilide; scrub and exfoliating particles such as polyethylene and silica or alumina; cooling agents such as menthol; skin calming agents such as aloe vera; and colorants.

In addition, the compositions of the invention may further include 0.5 to 10% by weight of sequestering agents, such as tetra sodium ethylenediaminetetraacetate (EDTA), EHDP or mixtures; opacifiers and pearlizers such as ethylene glycol distearate, titanium dioxide or Lytron 621 (Styrene/Acrylate copolymer); all of which are useful in enhancing the appearance or properties of the product.

The composition may comprise water in amount of 10 to 90% by weight of the composition, more preferably from 20 to 85%, even more preferably from 25 to 78%, most preferably from 32 to 65% by weight of the composition.

Preferably, the composition has a viscosity of at least 10 mPa s, more preferably in the range 30 to 10000 mPa s, even more preferably 50 to 5000 mPa s, and most preferably 100 to 2000 mPa s, when measured at 20 degrees C at a relatively high shear rate of about 20 s⁻¹. Preferably, the composition is in the form of fluid.

Preferably, the personal care composition is a skin cleansing composition. The term "skin" as used herein includes the skin on the face, neck, chest, abdomen, back, arms, under arms, hands, and legs. Preferably "skin" means includes the skin on the face and under arms, more preferably skin means skin on the face other than lips and eyelids.

The following examples are provided to facilitate an understanding of the invention. The examples are not intended to limit the scope of the claims.

### Examples

### Example 1

This example demonstrates the effect of the ratio of polyol to glycinate surfactant.

**Table 1**

| Samples (wt%)* | Ingredient | | | |
|---|---|---|---|---|
| | Water | Decyl Glucoside ^{a} | Sodium Cocoyl Glycinate ^{b} | Glycerin |
| A | To 100 | 3.00 | 2.88 | 0 |
| B | To 100 | 3.00 | 2.88 | 5.00 |
| C | To 100 | 3.00 | 2.88 | 10.00 |
| 1 | To 100 | 3.00 | 2.88 | 21.00 |
| D | To 100 | 0 | 6.16 | 10.00 |
| 2 | To 100 | 4.50 | 1.66 | 10.00 |
| 3 | To 100 | 6.00 | 0.16 | 10.00 |
| E | To 100 | 0 | 6.16 | 20.00 |
| F | To 100 | 1.50 | 4.66 | 20.00 |
| 4 | To 100 | 6.00 | 0.16 | 20.00 |
| 5 | To 100 | 4.79 | 1.37 | 23.56 |

| | | | | |
|---|---|---|---|---|
| *The level of the ingredients refers the level of active. a: PLANTACARE 2000UP, supplied by BASF b: Eversoft YCS-30S (U type), supplied by Sion lion | | | | |

A series of skin cleansing compositions were formulated according to Table 1 by following standard procedure.

The performances of the samples were evaluated by the procedure as follows: All samples were put into same bottles with pump. Identical amounts of samples were pumped out from the bottle onto watch glasses. Then, the water glasses with samples were placed into oven at 55°C for 24 hours. Then, these watch glasses were taken out, observed, and recorded in Table 2.

**Table 2**

| Sample | Weight ratio of Glycerin to glycinate | Score* |
|---|---|---|
| A | 0 | 5 |
| B | 1.74 | 5 |
| C | 3.47 | 4 |
| 1 | 7.29 | 0 |
| D | 1.62 | 5 |
| 2 | 6.02 | 1 |
| 3 | 62.5 | 0 |
| E | 3.25 | 5 |
| F | 4.29 | 4 |
| 4 | 125 | 0 |
| 5 | 16.8 | 0 |

| | | |
|---|---|---|
| *0: no residue; 1: rare residue; 2: slight residue; 3: noticeable residue; 4: obvious residue; 5: a lot of residue. | | |

It is surprisingly found that the residue was significantly decreasing by tuning the ratio of glycerin to the glycinate surfactant.

## Claims

1. A personal care composition comprising:
(a) glycinate surfactant;
(b) polyol; and
(c) nonionic surfactant comprising alkyl glucoside,
wherein:
(i) the weight ratio of the polyol to the glycinate surfactant is no less than 5.4:1;
(ii) the composition comprises phenoxyethanol; and
(iii) the total amount of the glycinate surfactant and alkyl glucoside is at least 2% by weight of the composition.

2. The composition according to claim 1 wherein the glycinate surfactant comprises alkyl glycinate, acyl glycinate or a mixture thereof, preferably the glycinate surfactant comprises C₁₂₋₂₀ alkyl glycinate, C₁₂₋₂₀ acyl glycinate, or a mixture thereof, more preferably the glycinate surfactant comprises C₁₂₋₂₀ acyl glycinate.

3. The composition according to claim 1 or 2 wherein glycinate surfactant is present in amount of 0.1 to 10%, preferably 0.5 to 6%, and more preferably 1.5 to 4% by weight of the composition.

4. The composition according any one of the preceding claims wherein the polyol is present in amount 6 to 40%, preferably 16 to 30% by weight of the composition.

5. The composition according any one of the preceding claims wherein weight ratio of the polyol to the glycinate surfactant is at least 6.3:1.

6. The composition according to any one of the preceding claims wherein the polyol comprises glycerin, propylene glycol, butylene glycol, polypropylene glycol, polyethylene glycol, sorbitol or a mixture thereof, preferably the polyol comprises glycerin.

7. The composition according to claim 5 wherein the composition comprises glycerin in amount 6 to 40%, preferably 16 to 30% by weight of the composition.

8. The composition according any one of claim 6 or 7 wherein weight ratio of the glycerin to the glycinate surfactant is at least 6.3:1.

9. The composition according to any one of the preceding claims wherein the nonionic surfactant comprises C₆ to C₂₀ alkyl glucoside.

10. The composition according to any one of the preceding claims wherein the nonionic surfactant is present in amount of 0.1 to 10%, preferably 1.5 to 4% by weight of the composition.

11. The composition according to any one of the preceding claims wherein the weight ratio of the glycinate surfactant to the nonionic surfactant is in the range of 1:8 to 8:1, preferably 1:2 to 2:1.

12. The composition according to any one of the preceding claims wherein the composition comprises water in amount of 10 to 90% preferably from 25 to 78% by weight of the composition.

13. The composition according to any one of the preceding claims wherein the composition is a skin cleansing composition.

14. Use of polyol to reduce the dried residue of a composition containing glycinate surfactant, wherein the weight ratio of the polyol to the glycinate surfactant is no less than 5.4:1.

## Patentansprüche

1. Körperpflegezusammensetzung, umfassend:
(a) Glycinat-Tensid;
(b) Polyol; und
(c) nicht-ionisches Tensid, umfassend Alkylglucosid,
wobei:
(i) das Gewichtsverhältnis des Polyols zum Glycinat-Tensid nicht weniger als 5,4:1 beträgt;
(ii) die Zusammensetzung Phenoxyethanol umfasst; und
(iii) die Gesamtmenge des Glycinat-Tensids und des Alkylglucosids mindestens 2 Gewichts-% der Zusammensetzung beträgt.

2. Zusammensetzung nach Anspruch 1, wobei das Glycinat-Tensid Alkylglycinat, Acylglycinat oder eine Mischung davon umfasst, wobei das Glycinat-Tensid vorzugsweise C₁₂₋₂₀-Alkylglycinat, C₁₂₋₂₀-Acylglycinat oder eine Mischung davon umfasst, bevorzugter das Glycinat-Tensid C₁₂₋₂₀-Acylglycinat umfasst.

3. Zusammensetzung nach Anspruch 1 oder 2, wobei das Glycinat-Tensid in einer Menge von 0,1 bis 10 Gewichts-%, vorzugsweise von 0,5 bis 6 Gewichts-% und bevorzugter von 1,5 bis 4 Gewichts-% der Zusammensetzung vorliegt.

4. Zusammensetzung nach irgendeinem der vorhergehenden Ansprüche, wobei das Polyol in einer Menge von 6 bis 40 Gewichts-%, vorzugsweise von 16 bis 30 Gewichts-% der Zusammensetzung vorliegt.

5. Zusammensetzung nach irgendeinem der vorhergehenden Ansprüche, wobei das Gewichtsverhältnis des Polyols zum Glycinat-Tensid mindestens 6,3:1 beträgt.

6. Zusammensetzung nach irgendeinem der vorhergehenden Ansprüche, wobei das Polyol Glycerin, Propylenglykol, Butylenglykol, Polypropylenglykol, Polyethylenglykol, Sorbitol oder eine Mischung davon umfasst, wobei das Polyol vorzugsweise Glycerin umfasst.

7. Zusammensetzung nach Anspruch 5, wobei die Zusammensetzung Glycerin in einer Menge von 6 bis 40 Gewichts-%, vorzugsweise von 16 bis 30 Gewichts-% der Zusammensetzung, umfasst.

8. Zusammensetzung nach irgendeinem der Ansprüche 6 oder 7, wobei das Gewichtsverhältnis des Glycerins zum Glycinat-Tensid mindestens 6,3:1 beträgt.

9. Zusammensetzung nach irgendeinem der vorhergehenden Ansprüche, wobei das nicht-ionische Tensid C₆- bis C₂₀-Alkylglucosid umfasst.

10. Zusammensetzung nach irgendeinem der vorhergehenden Ansprüche, wobei das nicht-ionische Tensid in einer Menge von 0,1 bis 10 Gewichts-%, vorzugsweise von 1,5 bis 4 Gewichts-% der Zusammensetzung vorliegt.

11. Zusammensetzung nach irgendeinem der vorhergehenden Ansprüche, wobei das Gewichtsverhältnis des Glycinat-Tensids zum nicht-ionischen Tensid in dem Bereich von 1:8 bis 8:1, vorzugsweise von 1:2 bis 2:1, liegt.

12. Zusammensetzung nach irgendeinem der vorhergehenden Ansprüche, wobei die Zusammensetzung Wasser in einer Menge von 10 bis 90 Gewichts-%, vorzugsweise von 25 bis 78 Gewichts-% der Zusammensetzung umfasst.

13. Zusammensetzung nach irgendeinem der vorhergehenden Ansprüche, wobei die Zusammensetzung eine Hautreinigungszusammensetzung ist.

14. Verwendung von Polyol zur Reduzierung des getrockneten Rückstands einer Zusammensetzung, die Glycinat-Tensid enthält, wobei das Gewichtsverhältnis des Polyols zum Glycinat-Tensid nicht weniger als 5,4:1 beträgt.

## Revendications

1. Composition pour le soin de la personne comprenant :
(a) un tensioactif de glycinate ;
(b) un polyol ; et
(c) un tensioactif non ionique comprenant un alkylglucoside,
dans laquelle :
(i) le rapport en masse du polyol au tensioactif de glycinate est d'au moins 5,4:1 ;
(ii) la composition comprend du phénoxyéthanol ; et
(iii) la quantité totale du tensioactif de glycinate et d'alkylglucoside est d'au moins 2 % en masse de la composition.

2. Composition selon la revendication 1, dans laquelle le tensioactif de glycinate comprend un glycinate d'alkyle, glycinate d'acyle ou un mélange de ceux-ci, de préférence le tensioactif de glycinate comprend un glycinate d'alkyle en C₁₂₋₂₀, glycinate d'acyle en C₁₂₋₂₀, ou un mélange de ceux-ci, encore mieux le tensioactif de glycinate comprend un glycinate d'acyle en C₁₂₋₂₀.

3. Composition selon la revendication 1 ou 2, dans laquelle le tensioactif de glycinate est présent dans une quantité de 0,1 à 10 %, de préférence 0,5 à 6 %, et encore mieux 1,5 à 4 % en masse de la composition.

4. Composition selon l'une quelconque des revendications précédentes, dans laquelle le polyol est présent dans une quantité de 6 à 40 %, de préférence 16 à 30 % en masse de la composition.

5. Composition selon l'une quelconque des revendications précédentes, dans laquelle le rapport en masse du polyol au tensioactif de glycinate est d'au moins 6,3:1.

6. Composition selon l'une quelconque des revendications précédentes, dans laquelle le polyol comprend de la glycérine, du propylène glycol, butylène glycol, polypropylène glycol, polyéthylène glycol, sorbitol ou un mélange de ceux-ci, de préférence le polyol comprend de la glycérine.

7. Composition selon la revendication 5, dans laquelle la composition comprend de la glycérine dans une quantité de 6 à 40 %, de préférence 16 à 30 % en masse de la composition.

8. Composition selon l'une quelconque de la revendication 6 ou 7, dans laquelle le rapport en masse de la glycérine au tensioactif de glycinate est d'au moins 6,3:1.

9. Composition selon l'une quelconque des revendications précédentes, dans laquelle le tensioactif non ionique comprend un alkylglucoside en C₆ à C₂₀.

10. Composition selon l'une quelconque des revendications précédentes, dans laquelle le tensioacif non ionique est présent dans une quantité de 0,1 à 10 %, de préférence 1,5 à 4 % en masse de la composition.

11. Composition selon l'une quelconque des revendications précédentes, dans laquelle le rapport en masse du tensioactif de glycinate au tensioactif non ionique se trouve dans l'intervalle de 1:8 à 8:1, de préférence 1:2 à 2:1.

12. Composition selon l'une quelconque des revendications précédentes, dans laquelle la composition comprend de l'eau dans une quantité de 10 à 90 %, de préférence de 25 à 78 % en masse de la composition.

13. Composition selon l'une quelconque des revendications précédentes, dans laquelle la composition est une composition nettoyante pour la peau.

14. Utilisation de polyol pour réduire le résidu séché d'une composition contenant un tensioactif de glycinate, dans laquelle le rapport en masse du polyol au tensioactif de glycinate est d'au moins 5,4:1.
